# EUROPEAN PATENT SPECIFICATION

(11) **EP 4 415 826 B1**
(45) Date of publication and mention of the grant of the patent: **09.09.2026**
(21) Application number: 22881487.7
(22) Date of filing: 27.09.2022
(51) Int. Cl.: A61P 35/00, C07D 253/10, A61K 31/53

(54) **SYNTHESIS AND IN VITRO ANTITUMOR ACTIVITIES OF NOVEL THIOAMIDE SUBSTITUTED PIPERAZINYL-1,2,4-TRIAZINES**
SYNTHESE UND IN VITRO ANTITUMORWIRKUNG VON THIOAMID SUBSTITUIERTEN PIPERAZINYL-1,2,4-TRIAZINEN
SYNTHÈSE ET ACTIVITÉS ANTITUMORALES IN VITRO DE NOUVELLES PIPÉRAZINYL-1,2,4-TRIAZINES SUBSTITUÉES PAR THIOAMIDE

(30) Priority: 15.10.2021 TR 202101614; 10.08.2022 TR 202201265
(43) Date of publication of application: 21.08.2024
(73) Proprietor: Istanbul Medipol Universitesi, Istanbul (TR)
(72) Inventor: BILTEKIN KALELI , Sevde Nur, Istanbul (TR); DEMIRAYAK, Seref, Istanbul (TR); SAHIN, Zafer, Istanbul (TR); BERK, Barkin, Istanbul (TR)
(74) Representative: Mutlu, Aydin
(86) International application number: PCT/TR2022/051044
(87) International publication number: WO 2023/063906

(56) References cited:
- YURTTAS LEYLA ET AL: "In vitro antitumor activity evaluation of some 1,2,4-triazine derivatives bearing piperazine amide moiety against breast cancer cells", BIOORGANIC & MEDICINAL CHEMISTRY, ELSEVIER, AMSTERDAM, NL, vol. 22, no. 22, 13 October 2014 (2014-10-13), pages 6313 - 6323, XP029091131, ISSN: 0968-0896, DOI: 10.1016/J.BMC.2014.10.002
- SAHIN ZAFER ET AL: "Abstract", vol. 59, no. 8, 6 March 2022 (2022-03-06), US, pages 1333 - 1340, XP093306843, ISSN: 0022-152X, Retrieved from the Internet <URL:https://onlinelibrary.wiley.com/doi/full-xml/10.1002/jhet.4470> DOI: 10.1002/jhet.4470
- EL-WAKIL MARWA H.; EL-YAZBI AMIRA F.; ASHOUR HAYAM M.A.; KHALIL MOUNIR A.; ISMAIL KHADIGA A.; LABOUTA IBRAHIM M.: "Discovery of a novel DNA binding agent via design and synthesis of new thiazole hybrids and fused 1,2,4-triazines as potential antitumor agents: Computational, spectrometric and in silico studies", BIOORGANIC CHEMISTRY, ACADEMIC PRESS INC., NEW YORK, NY., US, vol. 90, 26 June 2019 (2019-06-26), US , XP085760146, ISSN: 0045-2068, DOI: 10.1016/j.bioorg.2019.103089
- TOMáš GUCKý ; EVA ŘEZNíčKOVá ; PETR DžUBáK ; MARIáN HAJDúCH ; VLADIMíR: "Synthesis and anticancer activity of some 1,5-diaryl-3-(3,4,5-trihydroxyphenyl)-1H-pyrazolo¬4,3-e|¬1,2,4|triazines", MONATSHEFTE FÜR CHEMIE - CHEMICAL MONTHLY ; AN INTERNATIONAL JOURNAL OF CHEMISTRY, SPRINGER-VERLAG, AU, vol. 141, no. 6, 26 May 2010 (2010-05-26), AU , pages 709 - 714, XP019853484, ISSN: 1434-4475
- ABD EL-MONEIM MOHAMED, HASANEN J. A., EL-DEEN I. M., ABD EL-FATTAH W.: "Synthesis of fused 1,2,4-triazines as potential antimicrobial and antitumor agents", RESEARCH ON CHEMICAL INTERMEDIATES, AMSTERDAM, NL, vol. 41, no. 6, 1 June 2015 (2015-06-01), NL , pages 3543 - 3561, XP093061573, ISSN: 0922-6168, DOI: 10.1007/s11164-013-1470-z
- HOSNY MONA A., ZAKI YASSER H., MOKBEL WAFAA A., ABDELHAMID ABDOU O.: "Synthesis, Characterization, Antimicrobial Activity and Anticancer of Some New Pyrazolo[1,5-a]pyrimidines and Pyrazolo[5,1-c]1,2,4-triazines", MEDICINAL CHEMISTRY, BENTHAM SCIENCE PUBLISHERS LTD., NL, vol. 16, no. 6, 7 September 2020 (2020-09-07), NL , pages 750 - 760, XP093061579, ISSN: 1573-4064, DOI: 10.2174/1573406415666190620144404

## Description

### Technical Field

This invention is directed to novel thioamide substituted piperazinyl-1,2,4-triazines and their potential for use in treatment of malignant diseases, such as cancer.

### Prior Art

Nowadays, cancer treatment is still a complex and challenging process. An important ratio of people who was diagnosed with cancer, usually die in later stages. Treatment of cancer is made up of various methods such as radiotherapy, immunopherapy, surgery and drug therapy including small molecules. Brain tumors that originate from gliomas are malignant, aggresive and difficult to cure. There are only a few drugs such as temozolomide, cisplatin, kinase inhibitors or some of compounds affecting cAMP levels in treatment of brain tumors. None of these compounds are highly selective or potent, besides, it is not very clear which mechanistic pathway should be most focused in drug development for gliomas. Thus, there is need for development of new potent growth inhibitors of gliomas and glioblastomas.

### Detailed Description of the Invention

Present invention is directed to novel compounds with the general formula of Formula I shown below;

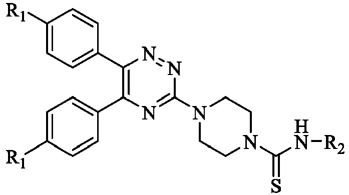

Herein R1 can be selected from a group comprising -H, -Cl, -OCH3 and R2 can be selected from a group comprising; -CH₂CH₃,

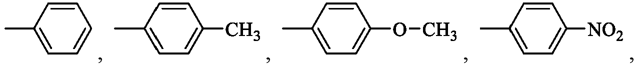

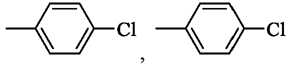

In an embodiment of the invention, the R1 groups as shown in formula I can be the same as selected from -H, -Cl, -OCH3 or the R1 groups shown in the formula I can be independently selected from the group of -H, -Cl, -OCH3. For example, while one R1 group is -H, the other R1 group can be -Cl etc.

In an embodiment of the invention; the compound according to present invention is named as formula Ia having the chemical structure shown below;

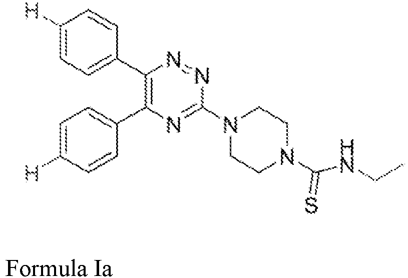

In an embodiment of the invention; the compound according to present invention is named as formula Ib having the chemical structure shown below;

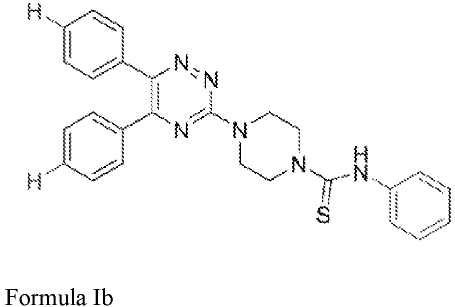

In an embodiment of the invention; the compound according to present invention is named as formula Ic having the chemical structure shown below;

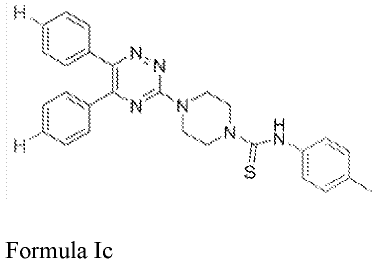

In an embodiment of the invention; the compound according to present invention is named as formula Id having the chemical structure shown below;

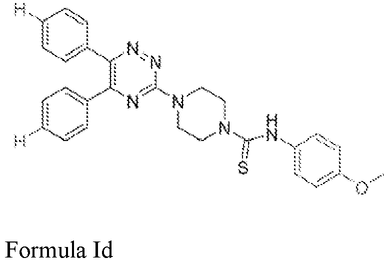

In an embodiment of the invention; the compound according to present invention is named as formula Ie having the chemical structure shown below;

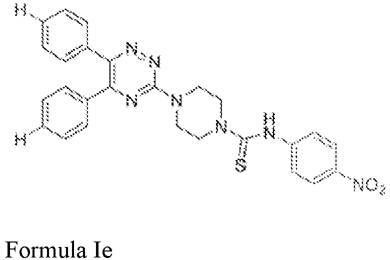

In an embodiment of the invention; the compound according to present invention is named as formula If having the chemical structure shown below;

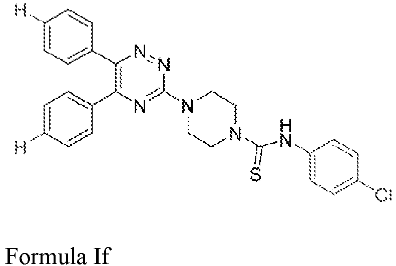

In an embodiment of the invention; the compound according to present invention is named as formula Ig having the chemical structure shown below;

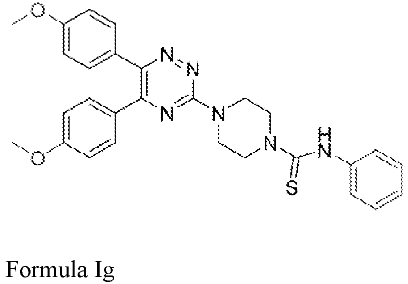

In an embodiment of the invention; the compound according to present invention is named as formula Ih having the chemical structure shown below;

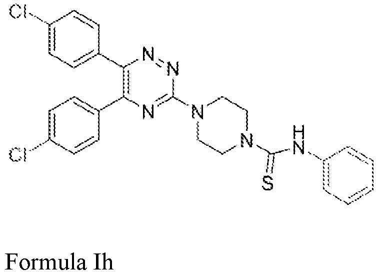

In an embodiment of the invention; the compound according to present invention is named as formula Ii having the chemical structure shown below;

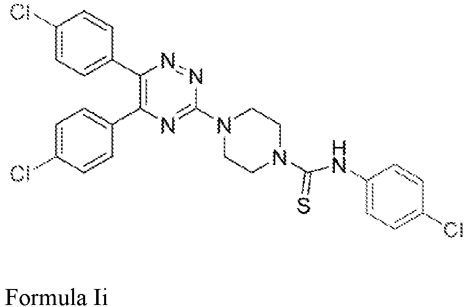

In a preferred embodiment of the invention, the compounds of Formula Ia-i according to present invention are shown below in Table 1 with respect to the R1 and R2 groups;

**Table 1: Examples of compounds according to formula I**

| 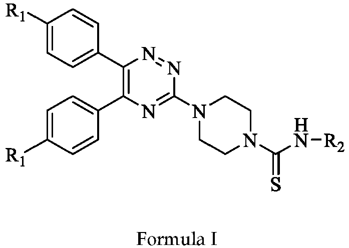 | | |
|---|---|---|
| Compound | R1 | R2 |
| Ia | H | 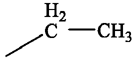 |
| Ib | H | 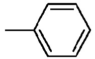 |
| Ic | H | 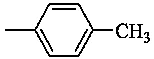 |
| Id | H | 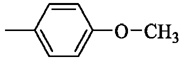 |
| Ie | H | 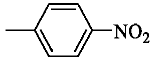 |
| If | H | 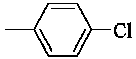 |
| Ig | OCH₃ | 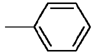 |
| Ih | Cl | 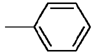 |
| Ii | Cl | 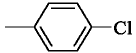 |

According to another aspect of the invention, the molecules shown with general Formula I, as described can be used in treating neoplastic diseases.

An aspect of the invention is directed to compounds of general formula I for use in treatment of neoplastic diseases
A preferred embodiment of the invention relates to compound of formula Ia or formula Ib or formula Ic or formula Id or formula Ie or formula If or formula Ig or formula Ih or formula Ii for use in treatment of neoplastic diseases.

The term "neoplastic diseases" used within the scope of the invention is directed to a physiological condition, for example, to cancer, which is characterized by malignant tumors or uncontrolled cell growth. The terms "neoplastic diseases" and "cancer" can be used interchangeably with each other. Cancer examples although not limited to these, comprise carcinomas, lymphomas, blastoma sarcomas, and leukemia.

Carcinoma, as used herein, expresses a type of cancer formed of epithelium cells.

Lymphoma, as used herein, describes a cancer type developed from lymphocytes.

Blastoma, as used herein, describes a cancer type progressing from precursor cells also known as blast cells.

Sarcoma, as used herein, refers to the type of cancer resulting from altered cells of mesenchymal origin.

Leukemia, as used herein, expresses the type of cancer that is induced in the bone marrow and causes the formation of the high number of abnormal white blood cells.

Specific examples related to cancer types comprises breast cancer, prostate cancer, colorectal cancer, skin cancer, small cell lung cancer, non-small cell lung cancer, mesothelioma, gastrointestinal cancer, pancreatic cancer, glioblastoma, vulva cancer, cervical cancer, endometrial carcinoma, ovarian cancer, liver cancer, hepatoma, bladder cancer, kidney cancer, salivary gland carcinoma, thyroid cancer and various head and neck cancers. A preferred embodiment of the invention is compound of Formula I or Formula Ia, Formula Ib, Formula Ic, Formula Id, Formula Ie, Formula If, Formula Ig, Formula Ih, Formula Ii for use in treatment of neuroblastoma, glioblastoma and/or breast cancer.

Compounds according to present invention were tested against C6 glioma, U87MG glioblastoma, MCF7 breast tumor, SH-SY5Y neuroblastoma, and HEK293 and NIH3T3 healthy cell lines.

The invention is furthermore related to a pharmaceutical composition comprising the compounds shown with general Formula I according to the invention.

The invention is furthermore related to a pharmaceutical composition comprising the compound of formula Ia or formula Ib or formula Ic or formula Id or formula Ie or formula If or formula Ig or formula Ih or formula Ii according to the invention.

Said pharmaceutical compositions can comprise at least one another active agent in addition to the compounds shown with general Formula I or in a preferred embodiment in addition to compound of formula Ia or formula Ib or formula Ic or formula Id or formula Ie or formula If or formula Ig or formula Ih or formula Ii according to the invention.

According to another embodiment of the invention the compounds of the invention shown with Formula I, can be used together with other active agents which have antineoplastic and/or cytotoxic and/or antimetastatic effects or with combinations comprising two/three of these agents. The second active agent can not only be formulated together with the compounds illustrated with Formula I, but it can also be sold in packages suitable to be used together after being formulated separately from the compound of Formula I.

According to another embodiment of the invention the compounds of the invention shown with formula Ia or formula Ib or formula Ic or formula Id or formula Ie or formula If or formula Ig or formula Ih or formula Ii according to the invention, can be used together with other active agents which have antineoplastic and/or cytotoxic and/or antimetastatic effects or with combinations comprising two/three of these agents. The second active agent can not only be formulated together with the compounds illustrated with formula Ia or formula Ib or formula Ic or formula Id or formula Ie or formula If or formula Ig or formula Ih or formula Ii according to the invention, but it can also be sold in packages suitable to be used together after being formulated separately from the compound of Formula I.

According to a preferred embodiment of the invention, the compounds illustrated with Formula I is combined with at least one active agent which has antineoplastic effects.

The active compounds which provide said antineoplastic effects that can be used in combination with the compounds illustrated with Formula I can be selected from the group comprised of cyclophosphamide, iphosphamide, temozolomide, kapecitabin, 5-fluoro uracil, methotrexate, gemcitabine, pemetrexed, mitomycin, bleomycin, epirubicin, doxorubicin, etoposide, paclitaxel, irinotecan, osdocetaxel, vincristin, opcarboplatin, cisplatin, ocaliplatin, setcabecab, dibabecab, dibabec, rituximab, sunitinib, zoledronate, abiraterone, anastrozole, bicalutamide, exemestane, goserelin, medroxyprogesterone, octreotide, tamoxifen, bendamustine, carmustine, chlorambucil, lomustine, melphalan, procarbazine, streptozotocin fludarabine, raltitrexed, actinomycin D, dactinomycin, doxorubycin, mitoxantrone, erybulin, topotecan, vinblastine, vinorelbine, afatinib, aflibersept, chrysotinib, dabrafenib, interferon, ipilimumab, lapatinib, nivolumab, panitumumab, pembrolizumab, pertuzumab, sorafenib, trastuzumab, emtansin, temsoril, vemurafenib, ibandronic acid, pamidronate, bexarotane, buserelin, Cyproterone, degareliks, folinic acid, fulvestrant, lanreotide, lenalidomide, letrozole, leuprorelin, megestrol, mesna, thalidomide or combinations comprising two or three of said agents.

According to another embodiment of the invention at least another active agent can be formulized together with or separately from the compounds illustrated with Formula I and at least said other active agents can have the same dosage, or can have a different dosage from the compounds of Formula I.

In the case that at least one other active agent mentioned above with the compounds of Formula I, is used, the other active agent, can be administered simultaneously, successively or sequentially with the agent(s) of Formula I.

In the case that at least one other active agent mentioned above with the compounds of formula Ia or formula Ib or formula Ic or formula Id or formula Ie or formula If or formula Ig or formula Ih or formula Ii is used, the other active agent can be administered simultaneously, successively or sequentially with the agents of formula Ia or formula Ib or formula Ic or formula Id or formula Ie or formula If or formula Ig or formula Ih or formula Ii.

The formulations according to the invention can comprise at least an excipient in addition to the active agent shown with general Formula I.

In a preferred embodiment of the invention, the formulations according to the invention can comprise at least an excipient in addition to the active agent shown with formula Ia or formula Ib or formula Ic or formula Id or formula Ie or formula If or formula Ig or formula Ih or formula Ii. The dosage range of the active agents that can be used together with Formula I shall be determined according to the requirements of the patient, the phase of the disease and the active agent to be used. The determination of a dose according to a specific situation is known by those that are skilled in the art.

The expression "comprising" within the scope of the invention is also used to mean "encompassing" .

Where it is technically suitable, the embodiments of the invention can be combined.

The embodiments have been described, such that they comprise the features/elements mentioned herein. The description essentially encompasses embodiments that comprise the mentioned features/elements or other embodiments constituted from these features/elements.

The clearly described specific embodiments can constitute a basis to waiver singularly or together with a few embodiments.

The invention will be further described below by referring to the examples that have been provided only for illustration and which should not be construed to limit the scope of the invention.

### Examples:

### Example 1: Chemical Synthesis of the compounds according to present invention

All starting materials, solvents and reagents were purchased from Sigma-Aldrich, VWR and . Infra-red spectrums were recorded in Perkin Elmer Spectrum Two instrument. ¹H and ¹³C NMR spectrums were recorded in Bruker 300- MHz UltraShield NMR Spectrophotometer (Bruker Corp, Billerica, Massachusetts, USA); and ¹³C NMR, Bruker 75- MHz UltraShield NMR Spectrophotometer (Bruker Corp, Billerica, Massachusetts, USA) in DMSO-d6 using standard TMS. All intermediate compounds were checked for their purities using thin-layer chromatography (TLC) with a Silica Gel 60 F254 TLC plate (Merck KGaA, Darmstadt, Germany). Melting points were measured by a Stuart Melting Point Apparatus SMP30 (Staffordshire, UK) and defined by recording the point where compound started to melt. The high resolution mass spectra of the compounds were determined on a Shimadzu 8040 LC/MS/MS ITTOF system (Shimadzu, Tokyo, Japan) using a mass spectrometer with the electron spray method (ESI).

### Example 2: Synthesis of benzil and bis 4-methoxy benzil derivatives

Aldehyde (1eq) was dissolved in absolute ethanol and 1.1 eq sodium cyanide added. Mixture was refluxed until the reaction was completed which takes approximately 2-3 h. Reaction was checked with TLC with the solvent system ethyl acetate 1:4 petroleum ether. Precipitated product was filtered off and washed with water and then ethanol for both derivatives. Crude product was recrystallized from ethanol. Obtained benzoine derivatives were then refluxed with ammonium nitrate, copper (II) acetate in AcOH to obtain benzil derivative. Benzil (**1a**) was checked for its melting point (95-96 °C) and was compatible with common literature data. 4-methoxybenzils (1,2-bis-(4-methoxyphenyl)ethanedione) (**1b**) melting point was measured as 130-133 °C as expected.

### Example 3: Synthesis of bis 4-chlorobenzil

Aldehyde (1eq) was dissolved in absolute ethanol and thiamine HCl (0.05 eq), TEA (0.3 eq) was added following Stetter procedure 24h. After completion, water was added and crude product precipitated. Recrystallized from ethanol to be used for next step.

### Example 4: Synthesis of 3-methylthio-5,6-diaryl-1,2,4-triazine derivatives

Following a previous paper, these derivatives (**2a-c**) were synthesized from benzils using S-methyl thiosemicarbazide HI salt [7]

### Example 5: Synthesis of 5,6-diaryl-3-(piperazine-1-yl)-1,2,4-triazine derivatives (3a-c)

As the product of previous step, S-methyl triazine derivative (1 eq) and piperazine (4 eq) were dissolved in pyridine and refluxed for 6h until the completion of methyl mercaptan odor. Reaction mixture was cooled, water added and resulting precipitate filtered. Then washed with water and dried. Recrystallized from ethanol to be used for the next step[7].

### Example 6: General synthesis of compounds Formula Ia-I

Piperazinyl triazine derivative from previous step (1 eq) were reacted with appropriate isothiocyanate derivative (1eq) in ethanol and refluxed for 6 h. Formed precipitation filtered and recrystallized from ethanol.

### 4-(5,6-diphenyl-1,2,4-triazin-3-yl)-N-ethylpiperazine-1-carbothioamide (Formula Ia)

Yield: 70% mp.: 236-238 °C. FT-IR υₘₐₓ (cm⁻¹): 3288.5 (N-H), 3053.1-2878.6 (C-H). ¹H NMR (300 MHz) DMSO-*d₆* δ (ppm): 1.13 (3H, t, *J:* 7.23 Hz, CH₃), 3.56 (2H, q, *J:* 5.29 Hz, CH₂), 3.98 (8H, brs, pip-CH₂), 7.24-7.29 (7H, m, Ar and NH), 7.39-7.48 (3H, m, Ar), 7.79 (1H, d, *J:* 4.94 Hz, Ar). ¹³C NMR (75 MHz) DMSO-*d₆* δ (ppm): 15.01 (CH₃), 40.62 (ethyl CH₂), 43.14 (piperazine CH₂), 46.82 (piperazine CH₂), 126.88, 127.18, 127.83, 128.73, 129.38, 129.90, 130.27, 130.69, 131.77, 136.57, 136.68, 148.93 (triazine C₆), 155.88 (triazine C₅), 159.60 (triazine C₃), 181.64 (C=S). C₂₂H₂₄N₆S.

### 4-(5,6-diphenyl-1,2,4-triazin-3-yl)-N-phenylpiperazine-1-carbothioamide (Formula Ib)

Yield: 75% mp.: 228-231 °C. FT-IR υₘₐₓ (cm⁻¹): 3244.6 (N-H), 3050-2874 (C-H). ¹H NMR (300 MHz) DMSO-*d₆* δ (ppm): 4.05 (4H, brs, pip-CH₂), 4.12 (4H, brs, pip-CH₂), 7.09-7.17 (1H, m, Ar), 7.26-7.41 (11H, m, Ar), 7.41-7.50 (3H, m, Ar), 9.45 (1H, s, NH). ¹³C NMR (75 MHz) DMSO-*d₆* δ (ppm): 43.21 (piperazine CH₂), 47.80 (piperazine CH₂), 124.90, 125.83, 128.52, 128.77, 129.40, 129.92, 130.32, 130.73, 136.58, 136.69, 141.40, 149.01 (triazine C₆), 155.95 (triazine C₅), 159.62 (triazine C₃), 182.07 (C=S). C₂₆H₂₄N₆S.

### 4-(5,6-diphenyl-1,2,4-triazin-3-yl)-N-(p-tolyl)piperazine-1-carbothioamide (Formula Ic)

Yield: 71% mp.: 226-227.5 °C. FT-IR υₘₐₓ (cm⁻¹): 3347.9 (N-H), 3100-2800 (C-H). ¹H NMR (300 MHz) DMSO-*d₆* δ (ppm): 2.28 (3H, s, CH₃), 4.04 (4H, brs, pip-CH₂), 4.11 (4H, brs, pip-CH₂), 7.12 (2H, d, *J:* 8.25 Hz, Ar), 7.20 (2H, d, *J:* 8.46 Hz, Ar), 7.32-7.40 (7H, m, Ar), 7.41-7.50 (3H, m, Ar), 7.41-7.50 (3H, m, Ar), 9.38 (1H, s, NH). ¹³C NMR (75 MHz) DMSO-*d₆* δ (ppm): 21.01 (CH₃), 43.21 (piperazine CH₂), 47.73 (piperazine CH₂), 126.04, 128.76, 129.00, 129.40, 129.92, 130.27, 130.72, 134.11, 136.58, 136.69, 138.80, 149.00 (triazine C₆), 155.93 (triazine C₅), 159.62 (triazine C₃), 182.14 (C=S). C₂₇H₂₆N₆S.

### 4-(5,6-diphenyl-1,2,4-triazin-3-yl)-N-(4-methoxyphenyl)piperazine-1-carbothioamide (Formula Id)

Yield: 64% mp.: 216-218 °C. FT-IR υₘₐₓ (cm⁻¹): 3346 (N-H), 3100-2800 (C-H). ¹H NMR (300 MHz) DMSO-*d₆* δ (ppm): 3.75 (3H, s, OCH₃), 4.05 (4H, brs, pip-CH₂), 4.12 (4H, brs, pip-CH₂), 6.89 (2H, dd, *J:* 8.81 Hz, 1.94 Hz, Ar), 7.23 (2H, dd, *J:* 8.81 Hz, 1.94 Hz, Ar), 7.28-7.55 (10H, m, Ar), 9.34 (1H, s, NH). ¹³C NMR (75 MHz) DMSO-*d₆* δ (ppm): 43.21 (piperazine CH₂), 47.63 (piperazine CH₂), 55.67 (OCH₃), 113.73, 127.95, 128.74, 129.40, 129.92, 130.71, 134.24, 136.58, 136.70, 148.97 (triazine C₆), 155.89 (triazine C₅), 157.01 (triazine C₃), 159.61 (Ar C-OCH₃), 182.30 (C=S). C₂₇H₂₆N₆OS.

### 4-(5,6-diphenyl-1,2,4-triazin-3-yl)-N-(4-nitrophenyl)piperazine-1-carbothioamide (Formula Ie)

Yield: 60% mp.: 232-235 °C. FT-IR υₘₐₓ (cm⁻¹): 3300.7 (N-H), 3100-2800 (C-H). ¹H NMR (300 MHz) DMSO-*d₆* δ (ppm): 4.08 (4H, brs, pip-CH₂), 4.15 (4H, brs, pip-CH₂), 7.32-7.40 (7H, m, Ar), 7.41-7.50 (3H, m, Ar), 7.67 (2H, d, *J:* 9.07 Hz, Ar), 8.18 (2H, dd, *J:* 7.09 Hz, 1.97 Hz, Ar), 9.97 (1H, s, NH). ¹³C NMR (75 MHz) DMSO-*d₆* δ (ppm): 43.18 (piperazine CH₂), 48.41 (piperazine CH₂), 123.12, 124.46, 128.77, 129.40, 129.91, 130.74, 136.55, 136.66, 142.60, 148.22, 149.07 (triazine C₆), 155.95 (triazine C₅), 159.58 (triazine C₃), 181.42 (C=S). C₂₆H₂₃N₇O₂S.

### N-(4-chlorophenyl)-4-(5,6-diphenyl-1,2,4-triazin-3-yl)piperazine-1-carbothioamide (Formula If)

Yield: 60% mp.: 222-225 °C. FT-IR υₘₐₓ (cm⁻¹): 3323.6 (N-H), 3100-2800 (C-H). ¹H NMR (300 MHz) DMSO-*d₆* δ (ppm): 4.06 (4H, brs, pip-CH₂), 4.12 (4H, brs, pip-CH₂), 7.33-7.41 (11H, m, Ar), 7.41-7.50 (3H, m, Ar), 9.51 (1H, s, NH). ¹³C NMR (75 MHz) DMSO-*d₆* δ (ppm): 43.18 (piperazine CH₂), 47.84 (piperazine CH₂), 127.36, 127.87, 128.38, 128.77, 129.40, 129.92, 130.73, 136.58, 136.68, 140.42, 149.03 (triazine C₆), 155.96 (triazine C₅), 159.61 (triazine C₃), 181.91 (C=S). C₂₆H₂₃ClN₆S.

### 4-(5,6-bis(4-methoxyphenyl)-1,2,4-triazin-3-yl)-N-phenylpiperazine-1-carbothioamide (Formula Ig)

Yield: 80% mp.: 193-196 °C. FT-IR υₘₐₓ (cm⁻¹): 3345 (N-H), 3100-2838.7(C-H). ¹H NMR (300 MHz) DMSO-*d₆* δ (ppm): 3.78 (6H, s, CH₃), 4.02 (4H, brs, pip-CH₂), 4.11 (4H, brs, pip-CH₂), 6.93 (2H, d, *J:* 5.90 Hz, Ar), 6.96 (2H, d, *J:* 5.81 Hz, Ar), 7.09-7.16 (1H, m, Ar), 7.27-7.37 (6H, m, Ar), 7.48 (2H, d, J: 8.88 Hz, Ar), 9.44 (1H, s, NH). ¹³C NMR (75 MHz) DMSO-*d₆* δ (ppm): 43.22 (piperazine CH₂), 47.84 (piperazine CH₂), 55.70 (OCH₃), 114.25, 114.34, 125.81, 128.51, 128.65, 129.29, 130.57, 131.66, 141.41, 148.61 (triazine C₆), 154.99 (triazine C₅), 159.46 (triazine C₃), 159.72 (Ar C-OCH₃), 161.44 (Ar C-OCH₃), 182.05 (C=S). C₂₈H₂₈N₆O₂S.

### 4-(5,6-bis(4-chlorophenyl)-1,2,4-triazin-3-yl)-N-phenylpiperazine-1-carbothioamide (Formula Ih)

Yield: 76% mp.: 220-222.8 °C. FT-IR υₘₐₓ (cm⁻¹): 3347.6 (N-H), 3000-2850 (C-H). ¹H NMR (300 MHz) DMSO-*d₆* δ (ppm): 4.06 (4H, brs, pip-CH₂), 4.11 (4H, brs, pip-CH₂), 7.09-7.16 (1H, m, Ar), 7.28-7.36 (4H, m, Ar), 7.37-7.53 (8H, m, Ar), 9.45 (1H, s, NH). ¹³C NMR (75 MHz) DMSO-*d₆* δ (ppm): 43.21 (piperazine CH₂), 47.77 (piperazine CH₂), 124.91, 125.82, 128.52, 129.01, 131.18, 131.84, 133.73, 135.23, 135.34, 135.81, 141.38, 147.76 (triazine C₆), 154.90 (triazine C₅), 159.55 (triazine C₃), 182.09 (C=S). C₂₆H₂₂Cl₂N₆S

### 4-(5,6-bis(4-chlorophenyl)-1,2,4-triazin-3-yl)-N-(4-chlorophenyl)piperazine-1-carbothioamide (Formula Ii)

Yield: 75% mp.: 191-197 °C. FT-IR υₘₐₓ (cm⁻¹): 3300 (N-H), 2868.5-3000 (C-H). ¹H NMR (300 MHz) DMSO-*d₆* δ (ppm): 4.06 (4H, brs, pip-CH₂), 4.12 (4H, brs, pip-CH₂), 7.35-7.40 (4H, m, Ar), 7.40-7.52 (8H, m, Ar), 9.50 (1H, s, NH). ¹³C NMR (75 MHz) DMSO-*d₆* δ (ppm): 43.17 (piperazine CH₂), 47.80 (piperazine CH₂), 127.36, 128.39, 128.81, 129.02, 131.18, 131.83, 133.74, 135.22, 135.33, 135.82, 140.40, 147.78 (triazine C₆), 154.91 (triazine C₅), 159.53 (triazine C₃), 181.91 (C=S). C₂₆H₂₁Cl₃N₆S

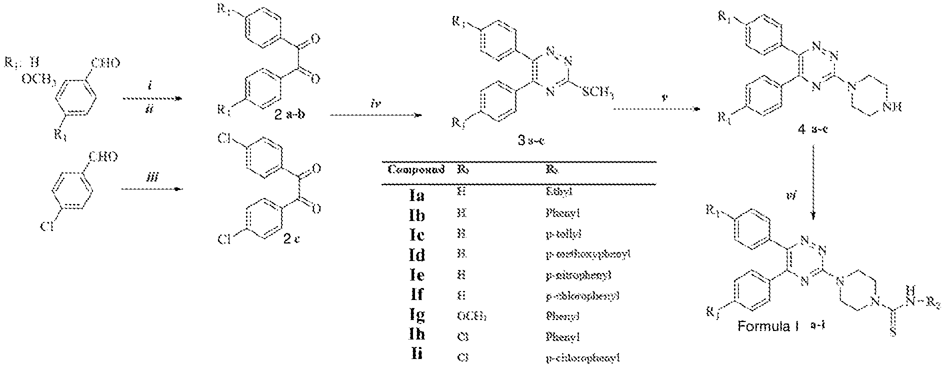

Scheme 1: Exemplary synthetic route for synthesis of compounds according to present invention (***i**:* 1 eq aldehyde, 1.1 eq sodium cyanide, ethanol, reflux 2h; ***ii**:* ammonium nitrate, cupper (II)acetate, benzoine derivative, AcOH reflux 4h; ***iii**:* thiamine HCl (0.05 eq), aldehyde (1 eq), ethanol, TEA (0.3 eq), R.T. 24h; ***iv**:* NaHCO₃, CH₃OH, reflux 3 h; v: pyridine, reflux 6h, ***vi**:* EtOH, reflux, 6h).

### Example 7: Biological activity tests

### Antitumor activity cell culture

In the study, four cell lines were purchased and used: SH-SY5Y(CRL-2266, ATCC, USA), C6 Glioma(CCL-107, ATCC, USA) and HEK293(CRL-1573, ATCC, USA). All cells were cultured and propagated in Dulbecco's Modified Eagle's Medium - high glucose (Cat. No:41966029, Gibco, USA), containing 10% (v/v) Foetal Bovine Serum (Cat. No:10500064, Gibco, USA), 10% (v/v) Penicillin-Streptomycin (10,000 U/mL)(Cat. No:15140122, Gibco, USA).

### MTT analysis

In order to assess the viability of the cells MTT (3-(4,5-Dimethylthiazol-2-yl)) assay was applied. Initially cells were seeded in the 96-well plates (VWR- 10062900) at a density of 10,000 cells/well and incubated in a cell culture incubator at 37 °C with 5% CO₂ for 24 hours. Then, the cells were incubated with the tested compounds at the concentration of 100µM, 50µM, 25µM and 12.5µM for 24 hours. After completion of incubation in molecules, all media were removed and 50 µl MTT (Gold Biotechnology T-030) solution was added to each well and the plates were incubated for an additional 3 hours, followed by addition of 150 µl of MTT solubilization mixture containing 0.1% Triton X-100 (Cat. No:A4025, Biomatik, USA ) and 4mM HCI (Cat. No:07102, Sigma-Aldrich, Germany) in isopropanol(Cat. No:278475, Sigma-Aldrich, Germany). The solution was slowly mixed for 15 minutes to dissolve the MTT formazan crystals and the absorbance of each well was measured with HIDEX Sense Microplate Reader(Cat. No:425-301, HIDEX, Finland) at the wavelength of 570 nm. Percent cytotoxicity and IC₅₀ values were calculated and the results are shown in table 2 below.

| | % inhibition (50 uM) | | | IC50 (uM) | | | SI |
|---|---|---|---|---|---|---|---|
| | SH-SY5Y | C6 | HEK293 | U87MG | MCF7 | NIH-3T3 | U87MG/NIH3T3, MCF7/NIH3T3 |
| 1a | 47.33 | 37.10 | 97.30 | 29.68 | 62.83 | 33.16 | 0.89, 1.89 |
| 1b | 84.90 | 86.10 | 96.10 | 45.98 | ND | 42.57 | 1.08, ND |
| 1c | 61.60 | 46.80 | 92.60 | ND | 73.12 | ND | ND, ND |
| 1d | 68.00 | 93.30 | 98.00 | 55.02 | 61.56 | ND | ND, ND |
| 1e | 21.90 | 94.40 | 95.90 | ND | ND | ND | ND, ND |
| 1f | 94.10 | 66.60 | 73.60 | 59.15 | ND | 65.32 | 0.90, ND |
| 1g | 46.20 | 63.70 | 97.40 | 68.12 | 91.15 | 76.03 | 0.89, 1.19 |
| 1h | 87.90 | 77.90 | 87.40 | ND | 77.16 | 40.17 | ND, 1.92 |
| 1i | 83.60 | 78.30 | 68.40 | 98.57 | ND | 81.53 | 1,21, ND |
| Cisplatin | 97.80 | 79.02 | 52.60 | 10.01 | 3.55 | 2.05 | 4.88, 1.73 |

### REFERENCES

[1] P.L. Bedard, D.M. Hyman, M.S. Davids, L.L. Siu, Small molecules, big impact: 20 years of targeted therapy in oncology, Lancet, 395 (2020) 1078-1088.
[2] K. Bukowski, M. Kciuk, R. Kontek, Mechanisms of Multidrug Resistance in Cancer Chemotherapy, Int J Mol Sci, 21 (2020).
[3] I.R. Whittle, D.M. Short, R.F. Deighton, L.E. Kerr, C. Smith, J. McCulloch, Proteomic analysis of gliomas, Br J Neurosurg, 21 (2007) 576-582.
[4] R. Sengupta, T. Sun, N.M. Warrington, J.B. Rubin, Treating brain tumors with PDE4 inhibitors, Trends Pharmacol Sci, 32 (2011) 337-344.
[5] P. Goldhoff, N.M. Warrington, D.D. Limbrick, Jr., A. Hope, B.M. Woerner, E. Jackson, A. Perry, D. Piwnica-Worms, J.B. Rubin, Targeted inhibition of cyclic AMP phosphodiesterase-4 promotes brain tumor regression, Clin Cancer Res, 14 (2008) 7717-7725.
[6] Z. Sahin, S.N. Biltekin, L. Yurttas, B. Berk, Y. Ozhan, H. Sipahi, Z.G. Gao, K.A. Jacobson, S. Demirayak, Novel cyanothiouracil and cyanothiocytosine derivatives as concentration-dependent selective inhibitors of U87MG glioblastomas: Adenosine receptor binding and potent PDE4 inhibition, Eur J Med Chem, 212 (2021) 113125.
[7] L. Yurttas, S. Demirayak, S. Ilgin, O. Atli, In vitro antitumor activity evaluation of some 1,2,4-triazine derivatives bearing piperazine amide moiety against breast cancer cells, Bioorg Med Chem, 22 (2014) 6313-6323.
[8] T.T. Kucukkilinc, K.S. Yanghagh, B. Ayazgok, M.A. Roknipour, F.H. Moghadam, A. Moradi, S. Emami, M. Amini, H. Irannejad, Synthesis and neuroprotective activity of novel 1,2,4-triazine derivatives with ethyl acetate moiety against H2O2 and A beta-induced neurotoxicity, Med Chem Res, 26 (2017) 3057-3071.
[9] F.E. Bleeker, R.J. Molenaar, S. Leenstra, Recent advances in the molecular understanding of glioblastoma, J Neuro-Oncol, 108 (2012) 11-27.
[10] F.E. Bleeker, S. Lamba, C. Zanon, R.J. Molenaar, T.J.M. Hulsebos, D. Troost, A.A. van Tilborg, W.P. Vandertop, S. Leenstra, C.J.F. van Noorden, A. Bardelli, Mutational profiling of kinases in glioblastoma, Bmc Cancer, 14 (2014).
[11] J. Pei, K.S. Moon, S. Pan, K.H. Lee, H.H. Ryu, T.Y. Jung, I.Y. Kim, W.Y. Jang, C.H. Jung, S. Jung, Proteomic Analysis between U87MG and U343MG-A Cell Lines: Searching for Candidate Proteins for Glioma Invasion, Brain Tumor Res Treat, 2 (2014) 22-28.
[12] S. Cascioferro, B. Parrino, V. Spano, A. Carbone, A. Montalbano, P. Barraja, P. Diana, G. Cirrincione, An overview on the recent developments of 1,2,4-triazine derivatives as anticancer compounds, Eur J Med Chem, 142 (2017) 328-375.
[13] S. Cascioferro, B. Parrino, V. Spano, A. Carbone, A. Montalbano, P. Barraja, P. Diana, G. Cirrincione, 1,3,5-Triazines: A promising scaffold for anticancer drugs development, Eur J Med Chem, 142 (2017) 523-549.
[14] M.K. Abou-Elregal, A.T.A. Mohamed, A.S.A. Youssef, M.M. Hemdan, S.S. Samir, W.S.I. Abou-Elmagd, Synthesis and antitumor activity evaluation of some 1, 2, 4-triazine and fused triazine derivatives, Synthetic Commun, 48 (2018) 2347-2357.

## Claims

1. An antineoplastic compound of Formula I 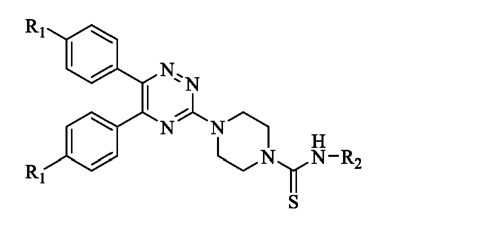 wherein;
• R1 is selected from a group comprising -H, -Cl, -OCH₃ and
• R2 is selected from a group comprising -CH₂CH₃, 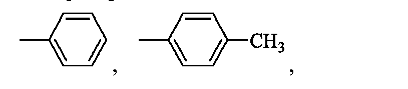 ,

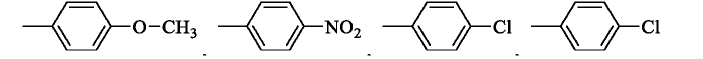

2. A compound according to claim 1, having the chemical structure of formula Ia 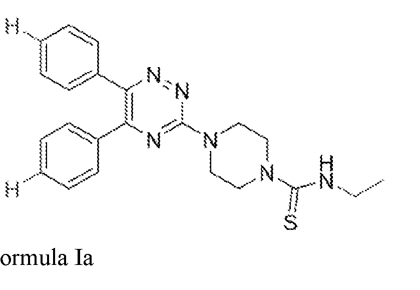 Formula Ia

3. A compound according to claim 1, having the chemical structure of formula Ib

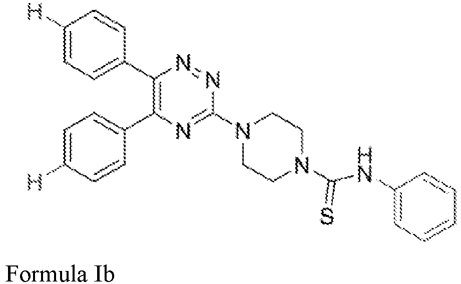

4. A compound according to claim 1, having the chemical structure of formula Ic

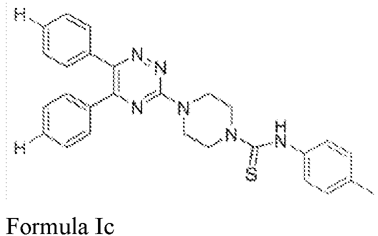

5. A compound according to claim 1, having the chemical structure of formula Id

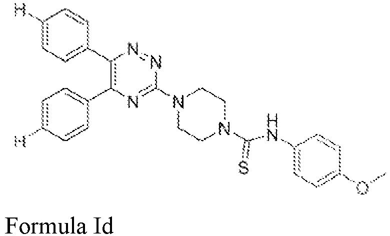

6. A compound according to claim 1, having the chemical structure of formula Ie

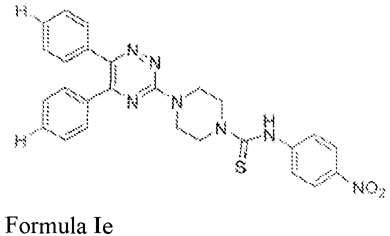

7. A compound according to claim 1, having the chemical structure of formula If

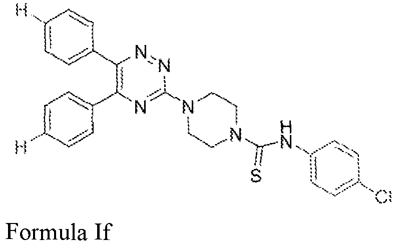

8. A compound according to claim 1, having the chemical structure of formula Ig

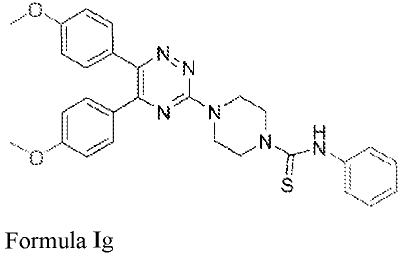

9. A compound according to claim 1, having the chemical structure of formula Ih

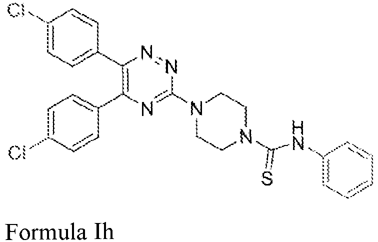

10. A compound according to claim 1, having the chemical structure of formula Ii

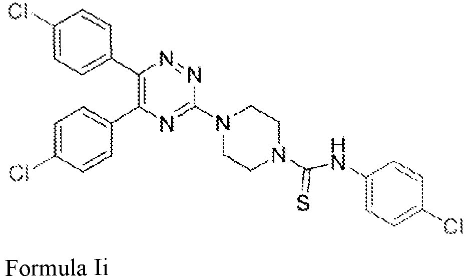

11. Compound of formula I according to any one of claims 1-10 for use in treatment of neoplastic diseases.

12. Compound of formula I for use according to claim 11, in treatment of breast cancer, prostate cancer, colorectal cancer, skin cancer, small cell lung cancer, non-small cell lung cancer, mesothelioma, gastrointestinal cancer, pancreatic cancer, glioblastoma, vulva cancer, cervical cancer, endometrial carcinoma, ovarian cancer, liver cancer, hepatoma, bladder cancer, kidney cancer, salivary gland carcinoma, thyroid cancer and various head and neck cancers.

13. Compound of formula I for use according to claim 11 or 12, in treatment of neuroblastoma, glioblastoma and/or breast cancer.

14. Pharmaceutical compositions comprising a compound of formula I according to any one of claims 1-10 as an active agent.

15. Pharmaceutical composition according to claim 14 further comprising at least one other active agent selected from antineoplastic agents, cytotoxic agents and/or antimetastatic agents

16. Pharmaceutical composition according to any one of claims 14 or 15 further comprising at least one pharmaceutically acceptable excipient.

## Patentansprüche

1. Antineoplastische Verbindung gemäß Formel I 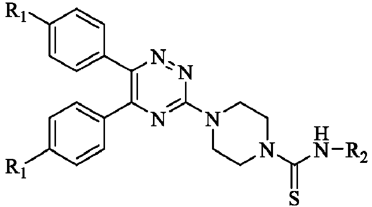 wobei:
• R₁ aus einer Gruppe ausgewählt ist, die -H, -Cl, -OCH₃ umfasst, und
• R₂ aus einer Gruppe ausgewählt ist, die Folgendes umfasst: -CH₂CH₃,

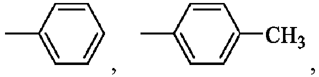

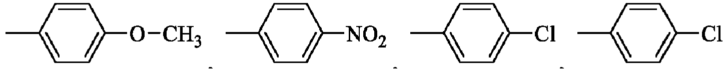

2. Verbindung nach Anspruch 1 mit der chemischen Struktur gemäß Formel Ia

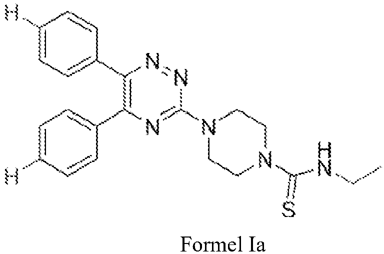

3. Verbindung nach Anspruch 1 mit der chemischen Struktur gemäß Formel Ib

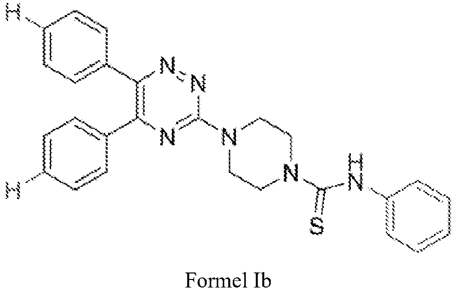

4. Verbindung nach Anspruch 1 mit der chemischen Struktur gemäß Formel Ic

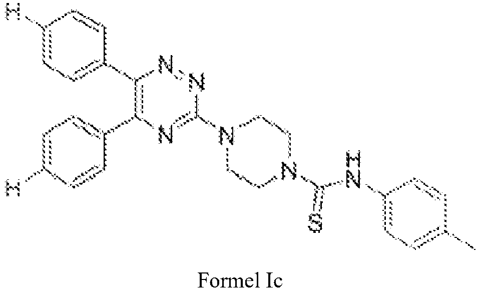

5. Verbindung nach Anspruch 1 mit der chemischen Struktur gemäß Formel Id

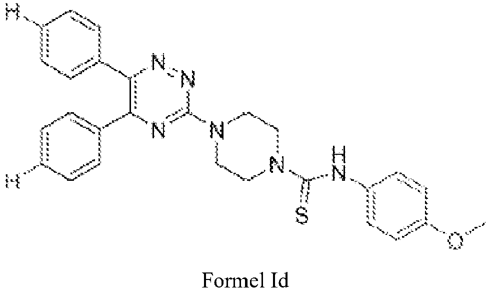

6. Verbindung nach Anspruch 1 mit der chemischen Struktur gemäß Formel Ie

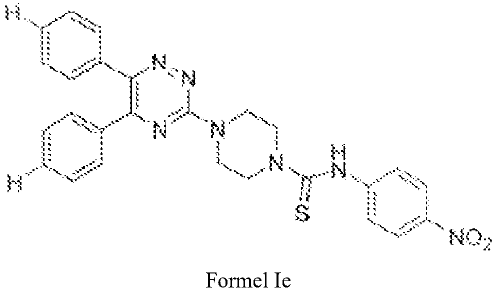

7. Verbindung nach Anspruch 1 mit der chemischen Struktur gemäß Formel If

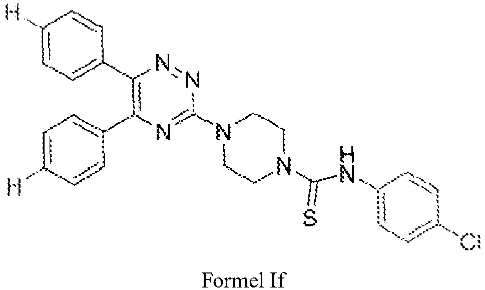

8. Verbindung nach Anspruch 1 mit der chemischen Struktur gemäß Formel Ig

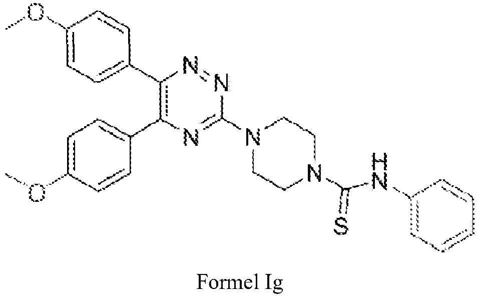

9. Verbindung nach Anspruch 1 mit der chemischen Struktur gemäß Formel Ih

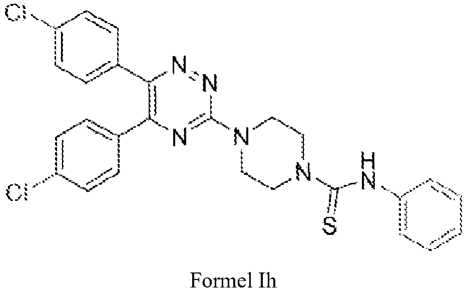

10. Verbindung nach Anspruch 1 mit der chemischen Struktur gemäß Formel Ii

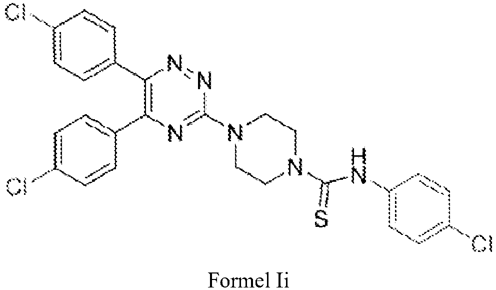

11. Verbindung gemäß Formel I nach einem der Ansprüche 1 bis 10 zur Verwendung bei der Behandlung von neoplastischen Erkrankungen.

12. Verbindung gemäß Formel I zur Verwendung nach Anspruch 11 bei der Behandlung von Brustkrebs, Prostatakrebs, Darmkrebs, Hautkrebs, kleinzelligem Lungenkrebs, nichtkleinzelligem Lungenkrebs, Mesotheliom, gastrointestinalem Krebs, Bauchspeicheldrüsenkrebs, Glioblastom, Vulvakrebs, Gebärmutterhalskrebs, Endometriumkarzinom, Eierstockkrebs, Leberkrebs, Hepatom, Blasenkrebs, Nierenkrebs, Speicheldrüsenkarzinom, Schilddrüsenkrebs sowie verschiedener Kopf- und Halskrebsarten.

13. Verbindung gemäß Formel I zur Verwendung nach Anspruch 11 oder 12 bei der Behandlung von Neuroblastom, Glioblastom und/oder Brustkrebs.

14. Pharmazeutische Zusammensetzungen, die eine Verbindung gemäß Formel I nach einem der Ansprüche 1 bis 10 als einen Wirkstoff enthalten.

15. Pharmazeutische Zusammensetzung nach Anspruch 14, die ferner mindestens einen weiteren Wirkstoff enthält, ausgewählt aus antineoplastischen Mitteln, zytotoxischen Mitteln und/oder antimetastatischen Mitteln.

16. Pharmazeutische Zusammensetzung nach einem der Ansprüche 14 oder 15, die ferner mindestens einen pharmazeutisch akzeptablem Hilfsstoff enthält.

## Revendications

1. Composé antinéoplasique de formule I 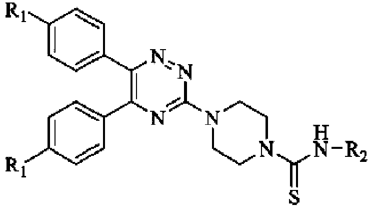 dans lequel :
• R1 est choisi dans le groupe comprenant -H, -Cl, -OCH₃ et
• R2 est choisi dans le groupe comprenant -CH₂CH₃,

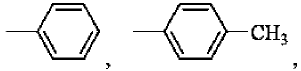

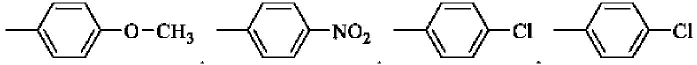

2. Composé selon la revendication 1, ayant la structure chimique de formule Ia

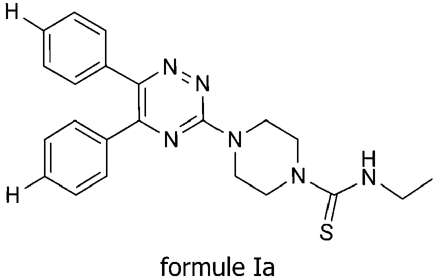

3. Composé selon la revendication 1, ayant la structure chimique de formule Ib

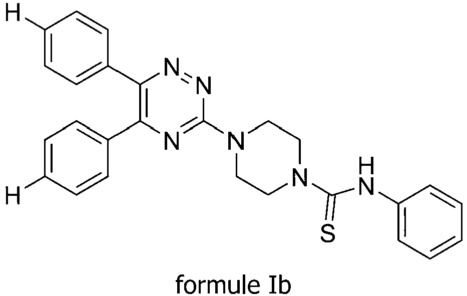

4. Composé selon la revendication 1, ayant la structure chimique de formule Ic

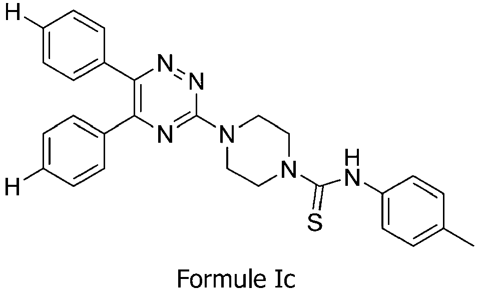

5. Composé selon la revendication 1, ayant la structure chimique de formule Id

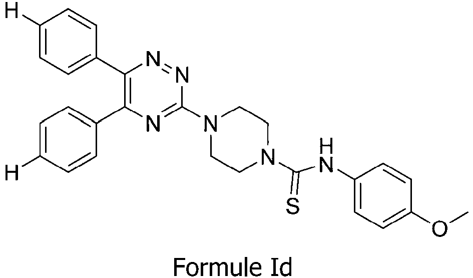

6. Composé selon la revendication 1, ayant la structure chimique de formule Ie

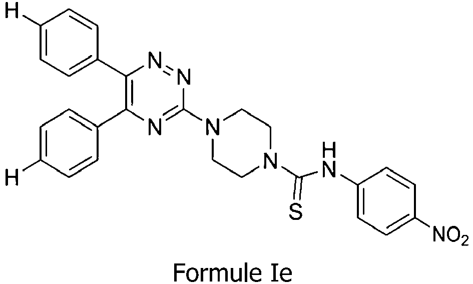

7. Composé selon la revendication 1, ayant la structure chimique de formule If

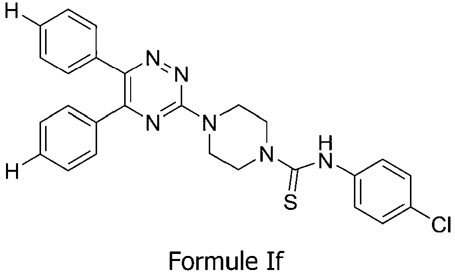

8. Composé selon la revendication 1, ayant la structure chimique de formule Ig

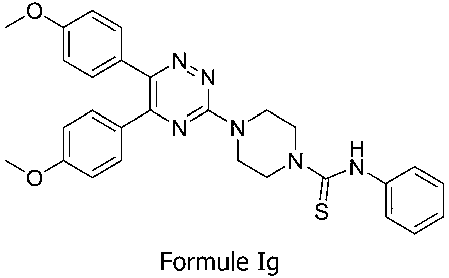

9. Composé selon la revendication 1, ayant la structure chimique de formule Ih

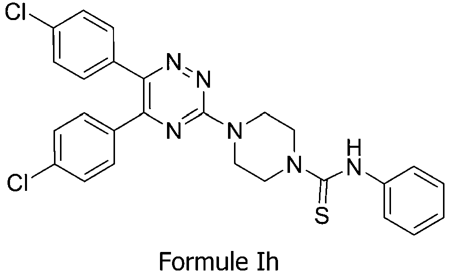

10. Composé selon la revendication 1, ayant la structure chimique de formule Ii

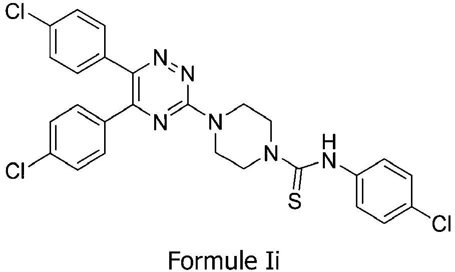

11. Composé de formule I selon l'une quelconque des revendications 1 à 10 pour une utilisation dans le traitement de maladies néoplasiques.

12. Composé de formule I pour une utilisation selon la revendication 11, dans le traitement d'un cancer du sein, d'un cancer de la prostate, d'un cancer colorectal, d'un cancer de la peau, d'un cancer du poumon à petites cellules, d'un cancer du poumon non à petites cellules, d'un mésothéliome, d'un cancer gastrointestinal, d'un cancer pancréatique, d'un glioblastome, d'un cancer de la vulve, d'un cancer du col, d'un carcinome endométrial, d'un cancer ovarien, d'un cancer du foie, d'un hépatome, d'un cancer de la vessie, d'un cancer du rein, d'un carcinome des glandes salivaires, d'un cancer de la thyroïde et de divers cancers de la tête et du cou.

13. Composé de formule I pour une utilisation selon la revendication 11 ou 12, dans le traitement d'un neuroblastome, d'un glioblastome et/ou d'un cancer du sein.

14. Compositions pharmaceutiques comprenant, en tant que principe actif, un composé de formule I selon l'une quelconque des revendications 1 à 10.

15. Composition pharmaceutique selon la revendication 14, comprenant en outre au moins un autre principe actif choisi parmi les agents antinéoplasiques, les agents cytotoxiques et/ou les agents antimétastatiques.

16. Composition pharmaceutique selon l'une quelconque des revendications 14 et 15, comprenant en outre au moins un excipient pharmaceutiquement acceptable.
